# EUROPEAN PATENT APPLICATION

(11) **EP 4 830 643 A1**
(43) Date of publication of application: **28.10.2026**
(21) Application number: 24908162.1
(22) Date of filing: 20.12.2024
(51) Int. Cl.: A61B 17/00

(54) **POWDER APPLICATOR**

(30) Priority: 21.12.2023 KR 20230188703
(71) Applicant: SAMYANG BIOPHARM CORPORATION, Seongnam-si Gyeonggi-do 13488 (KR)
(72) Inventor: KIM, Hyung Hwan, Seongnam-si Gyeonggi-do 13504 (KR); YOON, Hye Sung, Seongnam-si Gyeonggi-do 13544 (KR); LEE, Joo Hang, Seongnam-si Gyeonggi-do 13263 (KR)
(74) Representative: Gleiss Große Schrell und Partner mbB
(86) International application number: PCT/KR2024/020833
(87) International publication number: WO 2025/135880

(57) **Abstract**

The present invention relates to a powder applicator comprising: an air reservoir, which includes a first main body and a second main body that communicate with each other, forms the exterior of the powder applicator, and has both ends closed so as to be filled with air; a powder reservoir which is disposed between a discharge port through which powder is discharged and an operating part, and which includes a first sub-body that is disposed inside the first main body and includes a space in which the powder is to be filled, and an air supply passage that is formed at a portion adjacent to the discharge port of the first sub-body and includes at least one opening; a filter which is disposed, in correspondence to the air supply passage, between the powder reservoir and the air reservoir, and which blocks powder movement between the powder reservoir and the air reservoir and permits air movement therebetween; and the operating part that includes a plunger disposed inside the powder reservoir so as to divide the space of the first sub-body.

## Description

### [TECHNICAL FIELD]

The present invention relates to a powder applicator.

### [BACKGROUND ART]

During surgical operations, various bleeding situations occur. For bleeding with a large amount of bleeding, sutures, clips, staplers, etc. are used first. For additional small amounts of bleeding, sheets, gauze, powder, and gel-type hemostatic agents are used for the purpose of auxiliary hemostasis.

Unlike other types of hemostatic agents, powder-type hemostatic agents are used in an applicator, so they can be used for bleeding in deep and narrow areas that are difficult to reach with hands. In addition, since there are no shape restrictions, they have the advantage of being able to be used in various areas regardless of the bleeding site and area. However, since there are no shape restrictions, in order to accurately apply the powder hemostatic agent to the affected area, it must be used in a form filled in a powder spray applicator.

For these powder spray applicators, the amount used must be adjusted according to the amount of bleeding and the area of bleeding, so they must be able to spray multiple times, and the spray amount must be consistent so that the user can predict how much powder to use.

### [CONTENTS OF THE INVENTION]

### [PROBLEMS TO BE SOLVED]

The purpose of the present invention is to provide a powder applicator that is capable of spraying multiple times and at the same time has improved uniformity of powder spraying amount and accuracy of spraying timing.

The technical problems of the present invention are not limited to the technical problems mentioned above, and other technical problems that are not mentioned will be clearly understood by those skilled in the art to which the present invention belongs from the description below.

### [TECHNICAL MEANS]

The powder applicator according to the first aspect of the present invention comprises: an air reservoir comprising a first main body and a second main body interconnected with each other, forming the outline of a powder applicator, the two ends of which are closed and configured to contain air; a powder reservoir comprising a first sub-body and an air supply passage, wherein the first sub-body is disposed between a discharge port through which powder is discharged and an operating unit, is disposed inside the first main body, and includes a space configured to be filled with powder, and wherein the air supply passage is formed in a portion adjacent to the discharge port of the first sub-body and includes at least one opening; a filter disposed between the powder reservoir and the air reservoir so as to correspond to the air supply passage, wherein the filter is configured to block movement of powder between the powder reservoir and the air reservoir and to allow movement of air; and an operating unit including a plunger disposed inside the powder reservoir and dividing the space of the first sub-body.

In the powder applicator according to the second aspect of the present invention, the operating unit may further comprise a second sub-body that at least partially overlaps the powder reservoir and is slidably disposed with respect to the powder reservoir.

In the powder applicator according to the first aspect or the second aspect of the present invention, the operating unit may further comprise a spring disposed between the plunger and one end of the first sub-body to exert pressure on powder contained in the powder reservoir through the plunger.

In the powder applicator according to the first aspect or the second aspect of the present invention, the spring may have an elastic force for the plunger to be in close contact with one end surface of the internal space of the powder reservoir.

In the powder applicator according to the first aspect or the second aspect of the present invention, the plunger can be pressurized by the spring and can pressurize the powder so that there is no empty space in the internal space of the powder reservoir. The powder applicator according to the first aspect or the second aspect of the present invention may further comprise a barrier member coupled to the discharge port to block contact between the powder and the external environment.

In the powder applicator according to the first aspect or the second aspect of the present invention, the air reservoir may further comprise a handle portion having a structure protruding outwardly from the first main body or a handle portion formed as a separate structure from the first main body and coupled to one end of the first main body.

In the powder applicator according to the first aspect of the present invention, when an external force is applied to the second main body in one direction, the second main body may contract toward the first main body, the air filled in the air reservoir may increase in pressure so as to be supplied to the powder reservoir through the air supply passage, and the plunger may pressurize the powder filled in the powder reservoir.

In the powder applicator according to the first aspect of the present invention, the first main body and the second main body may be disposed to at least partially overlap, and the second main body may be in the form of a bellows member having a corrugated folding structure.

In the powder applicator according to the second aspect of the present invention, when an external force is applied to the second main body in one direction, the second main body and the second sub-body may move toward the first main body and the first sub-body, respectively, and the air filled in the air reservoir may increase in pressure so as to be supplied to the powder reservoir through the air supply passage, and the plunger may pressurize the powder filled in the powder reservoir.

In the powder applicator according to the second aspect of the present invention, the first main body and the second main body may be disposed to at least partially overlap, each of the first main body and the second main body may comprise a first stopping portion and a second stopping portion that are mutually engaged at end sides adjacent to each other, and the first main body and the second main body may overlap at least partially and may be slidable relative to each other.

In the powder applicator according to the second aspect of the present invention, the air reservoir may further comprise a bellows member, and the bellows member may be disposed between the first main body and the second main body and may have a structure that folds in a corrugated manner in one direction.

Specific details of other embodiments are included in the detailed description and drawings.

### [EFFECT OF THE INVENTION]

In the case of the powder applicator according to the embodiments of the present invention, the filled powder can be sprayed multiple times, and at the same time, the uniformity of the powder spray amount and the accuracy of the spray timing are improved.

The effects according to the embodiments of the present invention are not limited to the contents exemplified above, and more diverse effects are included in this specification.

### [Brief Description of the Figures]

Fig. 1 is a perspective view of a powder applicator according to one embodiment of the present invention.
Fig. 2 is a side view of a powder applicator according to one embodiment of the present invention.
Fig. 3 is a plan view of a powder applicator according to one embodiment of the present invention.
Fig. 4 is an exploded view of a powder applicator according to one embodiment of the present invention.
Figs. 5 to 7 are cross-sectional views of a powder applicator taken along line L-L' of Fig. 1, for explaining an operating method of a powder applicator according to one embodiment.
Figs. 8 to 10 are cross-sectional views for explaining an operating method of a powder applicator according to another embodiment of the present invention.
Figs. 11 to 13 are cross-sectional views for explaining an operating method of a powder applicator according to another embodiment of the present invention.
Fig. 14 is a schematic perspective view of a powder applicator according to another embodiment of the present invention.
Fig. 15 is a schematic perspective view of a powder applicator according to another embodiment of the present invention.
Fig. 16 is a cross-sectional view of the powder applicator of Fig. 15.
Fig. 17 is a perspective view showing an example in which a barrier member according to another embodiment of the present invention is applied to the powder applicator of Fig. 15.

### [CONCRETE MODE FOR CARRYING OUT THE INVENTION]

The advantages and features of the present invention, and methods for achieving them, will become apparent upon reference to the embodiments described in detail with reference to the accompanying drawings. However, the invention is not limited to the embodiments disclosed herein, but will be embodied in many different forms, and these embodiments are provided only to make the disclosure of the present invention complete and to fully inform a person having ordinary skill in the art to which the present invention belongs of the scope of the invention, and the present invention is defined only by the scope of the claims.

Throughout the specification, like reference numerals refer to like components. The shapes, sizes, proportions, angles, numbers, etc. disclosed in the drawings to illustrate embodiments are exemplary and do not limit the invention to those shown.

Although the terms first, second, and the like are used to describe various components, the components are not limited by these terms. Thus, a first component referred to herein may also be a second component within the technical idea of the present invention.

The individual features of the various embodiments of the present invention may be partially or wholly combined or combined with each other, and various technical connections are possible, and the embodiments may be implemented independently of each other or implemented together in a related relationship.

Specific embodiments will be described below with reference to the accompanying drawings.

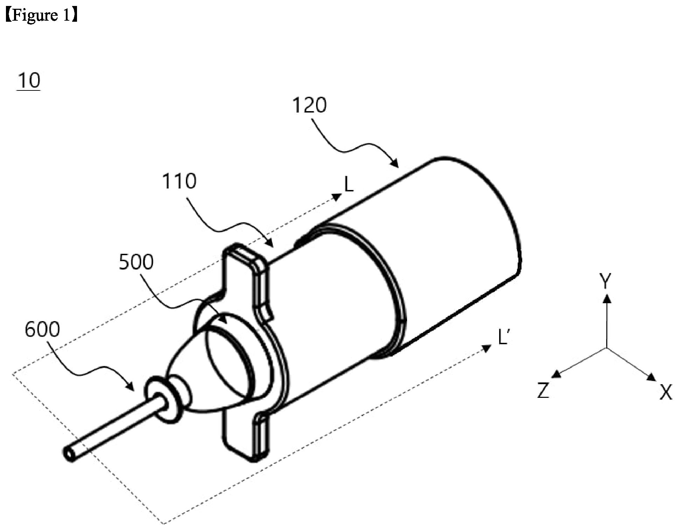

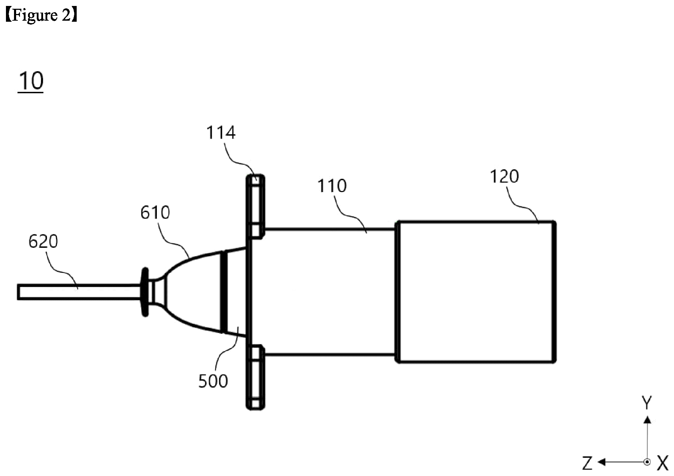

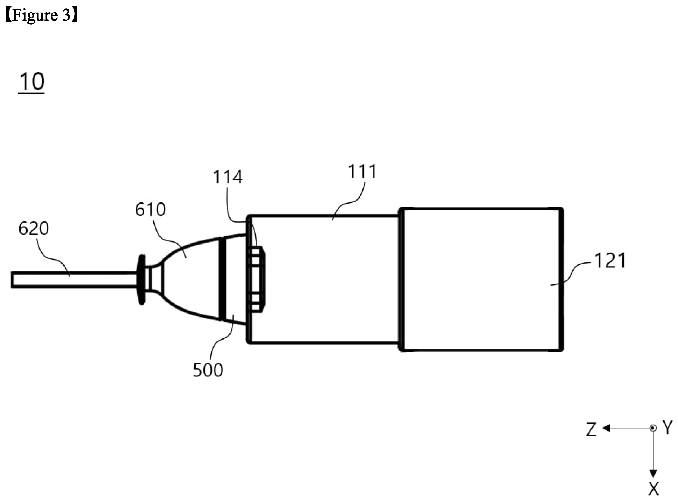

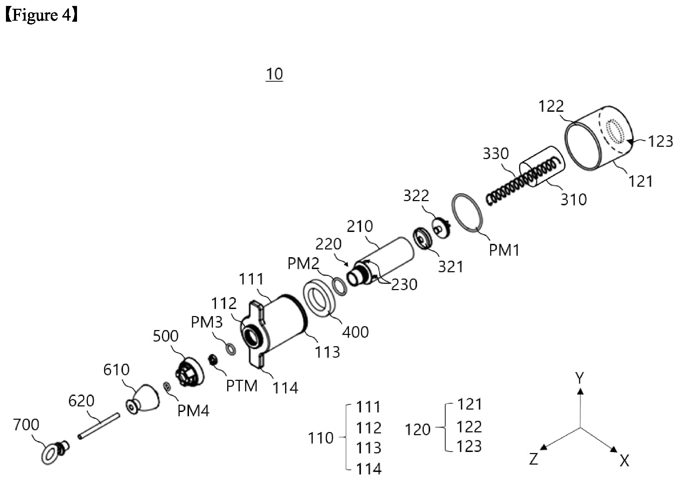

Fig. 1 is a perspective view of a powder applicator according to one embodiment of the present invention. Fig. 2 is a side view of a powder applicator according to one embodiment of the present invention. Fig. 3 is a plan view of a powder applicator according to one embodiment of the present invention. Fig. 4 is an exploded view of a powder applicator according to one embodiment of the present invention.

First, in order to more clearly explain the technical idea of the present invention, a coordinate system set in a first direction (X), a second direction (Y) perpendicular to the first direction (X) and in the same plane, and a third direction (Z) perpendicular to the planes of the first direction (X) and the second direction (Y) is described. The first direction (X), the second direction (Y), and the third direction (Z) are intended to explain directions related to the structure and operation method of the powder applicator (10), and should be understood to mean relative directions, and the embodiments are not limited to the mentioned directions.

For example, when viewed from the front, the first direction (X) may mean the horizontal direction of the powder applicator (10), the second direction (Y) may mean the vertical direction of the powder applicator (10), and the third direction (Z) may mean the height direction of the powder applicator (10).

Referring to Figs. 1 to 4, a powder applicator (10) according to one embodiment may comprise an air reservoir (100), a powder reservoir (200), an operating unit (300), a filter (400), and a discharge port (500), and may optionally additionally comprise a spray member (600) and/or a barrier member (700). In addition, the powder applicator (10) may further comprise a plurality of sealing members (PM1, PM2, PM3, PM4) to provide airtightness between each component.

For example, the powder applicator (10) may comprise an air reservoir (100) that forms the main outer shape of the powder applicator (10) and has a part of its internal space configured to contain air, a powder reservoir (200) that is disposed in the internal space of the air reservoir (100) and filled with powder, an operating unit (300) that slides in the third direction (Z) to the powder reservoir (200) when pressurized by an external force and pressurizes the powder filled in the powder reservoir (200) and slides in the opposite direction of the third direction (Z) when the external force is released, a filter (400) that is disposed between the powder reservoir (200) and the air reservoir (100) and configured to allow the flow of air and to block the flow of powder, a discharge port (500) through which powder filled in the powder reservoir (200) is discharged by the air pressure, a spray member (600) that is mounted on the discharge port (500) and sprays powder discharged from the discharge port (500), and a barrier member (700) that is mounted on the discharge port (500) instead of the spray member (600) to block powder from being discharged.

The air reservoir (100) may comprise a first main body (110) and a second main body (120). The air reservoir (100) may have a structure in which the first main body (110) is positioned at the top and the second main body (120) is positioned at the bottom in the height direction (Z). The first main body (110) and the second main body (120) may slide relative to each other in the third direction (Z) by an external force applied in the third direction (Z).

The first main body (110) and the second main body (120) may be aligned along the third direction (Z) and may be disposed so that at least a portion thereof overlaps. The first main body (110) and the second main body (120) may have a cylindrical shape, but are not limited thereto. For example, the first main body (110) and the second main body (120) can have an elliptical shape or a polygonal shape such as a triangle, a square, a pentagon, etc. on the X-Y plane.

For example, the first main body (110) may be entirely transparent or partially transparent. In addition, the first main body (110) may be a shape with a constant diameter or a tapered shape formed so that the width in the third direction (Z) changes. In addition, the second main body (120) may be a bellows member, and may be a shape with a constant diameter or a tapered bellows member formed so that the width in the third direction (Z) changes.

The first main body (110) and the second main body (120) may be formed so that one has a larger area in the X-Y plane than the other, and accordingly, they may be disposed so that one having a larger area in the plane has a shape that surrounds at least a portion of the outer circumference of the other.

For example, if the first main body (110) and the second main body (120) have a cylindrical shape and the diameter of the second main body (120) is implemented to be larger than the diameter of the first main body (110), the upper part of the second main body (120) may have a shape that surrounds the lower part of the first main body (110). A first sealing member (PM1) may be disposed in the portion where the first main body (110) and the second main body (120) overlap. The first sealing member (PM1) may be an O-ring for airtightness of the portion where the first main body (110) and the second main body (120) overlap.

However, it is not limited thereto, and the diameter of the first main body (110) may be implemented to be larger than the diameter of the second main body (120), and the lower part of the first main body (110) may have a shape that surrounds the upper part of the second main body (120), in which case, as can be understood by a person skilled in the art, some features (e.g., the positions and sizes of the first stopping portion (113), the second stopping portion (122), and the first sealing member (PM1)) may be modified.

The first main body (110) may comprise a first body portion (111) and a first coupling portion (112), and may optionally further comprise a first stopping portion (113) and a handle portion (114). The first body portion (111) may constitute the main shape of the first main body (110). The first coupling portion (112) has a structure to which a discharge port (500) is coupled on an outer surface, and may be formed on one end side of the first body portion (111) in the third direction (Z). For example, the first coupling portion (112) may have a shape protruding from the first body portion (111) in the third direction (Z), and may have a structure to be coupled with the discharge port (500).

As a specific example, the first coupling portion (112) may have screw threads formed on the outer surface to be coupled with the discharge port (500) and screw threads formed on the inner surface to be coupled with the powder reservoir (200), but is not limited thereto, and the structure of the first coupling portion (112) may be variously modified depending on the structures of the discharge port (500) and the powder reservoir (200).

The handle portion (114) may have a structure that supports the powder applicator (10) when the user uses the powder applicator (10) and an external force is applied in the third direction (Z). The handle portion (114) may have a structure that protrudes from the side of the first body portion (111) or may be formed as a separate configuration from the first body portion (111) and coupled to one end of the first body portion (111).

For example, the handle portion (114) may have a shape of the number 1 that protrudes in the second direction (Y) and the opposite direction of the second direction (Y) from the side of the first body portion (111) adjacent to the first coupling portion (112) and crosses the first body portion (111) when viewed in the third direction (Z), and the portions protruding in the second direction (Y) and the opposite direction of the second direction (Y) may be symmetrical or asymmetrical to each other. However, the shape of the handle portion (114) is not limited thereto, and may have various shapes for a user to support the powder applicator (10) in the third direction (Z).

The first stopping portion (113) may be formed on the other end side of the first body portion (111) in the third direction (Z). The first stopping portion (113) may have a shape in which the other end of the first body portion (111) protrudes outward along the side periphery. The first stopping portion (113) is implemented to engaged with the second stopping portion (122) of the second main body (120), thereby preventing the first main body (110) and the second main body (120) from being separated in the third direction (Z). The degree of protrusion of the first stopping portion (113) may be determined according to the difference in diameters between the first main body (110) and the second main body (120).

In the present embodiment, since the diameter of the second body portion (121) is exemplified as being larger than the diameter of the first body portion (111), the first stopping portion (113) is exemplified as protruding outwardly of the first body portion (111). However, if the diameter of the first body portion (111) is larger than the diameter of the second body portion (121), the first stopping portion (113) may have a structure protruding inwardly of the first body portion (111). In this case, as will be described later, the second stopping portion (122) may have a structure protruding outwardly of the second body portion (121).

The second main body (120) comprises a second body portion (121) and may optionally further comprise a second stopping portion (122) and a support structure (123). The second body portion (121) may constitute a main shape of the second main body (120). The second stopping portion (122) and the support structure (123) may be formed on the second body portion (121).

The second stopping portion (122) may be formed on one end side of the second body portion (121) in the third direction (Z). The second stopping portion (122) may have a shape in which one end of the second body portion (121) protrudes inwardly along the side periphery. The second stopping portion (122) may be implemented to be interlocked with the first stopping portion (113). The second stopping portion (122) may be deformed according to the structure of the first stopping portion (113).

The support structure (123) may be formed on the inner surface of the other end of the second body portion (121) in the third direction (Z). The support structure (123) may be a structure to which the operating unit (300) is fixed. For example, the support structure (123) may protrude inwardly (in the third direction (Z)) from the other end surface of the second body portion (121) and may have an O-shape when viewed from the third direction (Z). The outer diameter of the support structure (123) may be set so that the second sub-body (310) of the operating unit (300) is interlocked and fixed. In addition, the inner diameter of the support structure (123) may be set so that the spring (330) of the operating unit (300) is disposed inside it.

The first main body (110) and the second main body (120) of the air reservoir (100) form the main outer shape of the powder applicator (10), and a powder reservoir (200) and an operating unit (300) can be disposed inside the air reservoir (100). A space other than the space where the powder reservoir (200) and the operating unit (300) are disposed among the internal spaces of the air reservoir (100) can be configured to contain air.

According to one embodiment of the present invention, the internal space of the air reservoir (100) may have its volume reduced as the first main body (110) and the second main body (120) slide relative to each other due to an external force applied in the third direction (Z), thereby increasing the pressure of the air filled in the internal space.

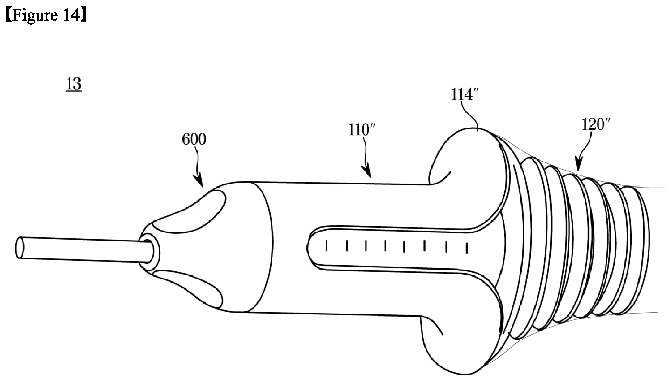

According to another embodiment of the present invention, as illustrated in Figs. 14 to 17, when an external force is applied to the second main body (120" or 120‴) in one direction, the second main body (120" or 120‴) contracts toward the first main body (110" or 110‴), the air filled in the air reservoir (100) increases in pressure so as to be supplied to the powder reservoir (200) through the air supply passage, and the plunger (320) can pressurize the powder filled in the powder reservoir (200). For example, the first main body (110" or 110‴) and the second main body (120" or 120‴) may be disposed to overlap at least partially, and the second main body (120" or 120‴) may have a bellows member shape having a structure that folds in a corrugated manner.

The powder reservoir (200) may comprise a first sub-body (210) disposed inside the first main body (110), a second coupling portion (220) formed at one end of the first sub-body (210) in the third direction (Z), and an air supply passage (230) disposed on the side of the first sub-body (210) adjacent to the second coupling portion (220).

The X-Y plane shape of the first sub-body (210) may be cylindrical, but is not limited thereto. For example, the X-Y plane shape of the first sub-body (210) may have an elliptical shape or a polygonal shape such as a triangle, square, or pentagon.

The first sub-body (210) may have a smaller diameter than the first main body (110). As a non-limiting example, the width of the first sub-body (210) in the third direction (Z) may be larger than the width of the first main body (110) in the third direction (Z). The first sub-body (210) may be configured to be filled with powder therein.

The second coupling portion (220) may have a structure protruding from one end of the first sub-body (210) in the third direction (Z). For example, the second coupling portion (220) may have a cylindrical shape with a diameter smaller than that of the first sub-body (210), and at least a portion of the outer peripheral surface thereof may have a coupling structure for coupling with the inner peripheral surface of the first coupling portion (112) of the first main body (110).

For example, the diameter of the outer surface of the second coupling portion (220) corresponds to the diameter of the inner surface of the first coupling portion (112), and screw threads may be formed on at least a portion of the outer surface of the second coupling portion (220) to be screw-coupled by engaging with the screw threads of the inner surface of the first coupling portion (112). In addition, a second sealing member (PM2) may be disposed at a portion where the outer surface of the second coupling portion (220) and the inner surface of the first coupling portion (112) come into contact to ensure airtightness between the two components. The second sealing member (PM2) may be an O-ring having a size corresponding to the outer surface of the second coupling portion (220).

A discharge port (500) may be fitted into the inner surface of the second coupling portion (220). In some embodiments, a coupling structure may be formed on the inner surface of the second coupling portion (220) to which the discharge port (500) is coupled.

The air supply passage (230) may comprise at least one opening formed on a side surface of one end of the first sub-body (210). For example, the air supply passage (230) may comprise four openings disposed along the perimeter of the first sub-body (210). However, the present invention is not limited thereto, and the air supply passage (230) may comprise three or fewer, or five or more openings. In addition, the opening comprised in the air supply passage (230) may have a circular shape in a plan view, but is not limited thereto, and may be formed in an elliptical shape or a polygonal shape such as a triangle, a square, a pentagon, etc.

The powder reservoir (200) can be interconnected with the air reservoir (100) through the air supply passage (230). The powder filled in the powder reservoir (200) can be discharged to the discharge port (500) by the compressed air introduced through the air supply passage (230). As described below, the powder applicator (10) according to the present invention can be advantageous in minimizing air resistance by the powder trap (PT) and efficiently injecting compressed air into the powder reservoir (200) to discharge the powder filled in the powder reservoir (200) since the air supply passage (230) is implemented on the side adjacent to the discharge port (500) and the powder trap (PT).

The operating unit (300) may comprise a plunger (320) and optionally further comprise a second sub-body (310) and a spring (330). The second sub-body (310) may have a shape corresponding to the first sub-body (210) in order to at least overlap with it. Accordingly, the second sub-body (310) may have a cylindrical shape, but is not limited thereto, and like the first sub-body (210), the shape on the X-Y plane may have an elliptical or polygonal shape such as a triangle, a square, a pentagon, etc.

The second sub-body (310) can be disposed inside the second main body (120). The second sub-body (310) can be disposed to overlap at least partially with the powder reservoir (200), such that the first sub-body (210) and the second sub-body (310) may slide over each other in the third direction (Z).

An upper portion (one end side in the third direction (Z)) of the second sub-body (310) may at least partially overlap with a lower portion of the first sub-body (210). The diameter of the second sub-body (310) may be larger than a diameter of the first sub-body (210). For example, by forming a diameter of the inner surface of the second sub-body (310) to correspond to a diameter of the outer surface of the first sub-body (210), the inner surface of the second sub-body (310) may surround an outer surface of the first sub-body (210).

In addition, the diameter of the second sub-body (310) may be set to be fixed by the support structure (123). For example, the diameter of the second sub-body (310) may be formed such that the inner surface corresponds to the outer surface of the support structure (123). In this case, the diameter of the support structure (123) and the diameter of the outer surface of the first sub-body (210) may be substantially the same, but are not limited thereto.

The second sub-body (310) may be fitted and connected to the outer surface of the support structure (123). However, the present invention is not limited thereto, and as another example, a structure for fixing each other, for example, a screw thread for screw connection, may be formed on the other end of the second sub-body (310) and the outer surface of the support structure (123), respectively. In some embodiments, the second sub-body (310) may be formed integrally with the second main body (120), in which case the support structure (123) may be omitted. In addition, the width of the second sub-body (310) in the third direction (Z) may be smaller than the width of the second main body (120) in the third direction (Z), but is not limited thereto.

The size difference in width in the third direction (Z) of each of the first main body (110), the second main body (120), the first sub-body (210), and the second sub-body (310) and the width between each of the components may be determined according to the amount of powder filled in the powder reservoir (200), the size and elastic coefficient of the spring (330), the air pressure required to discharge the powder filled in the powder reservoir (200), etc.

Likewise, the diameter of each of the first main body (110), the second main body (120), the first sub-body (210), and the second sub-body (310) can be designed according to the amount of powder filled in the powder reservoir (200), the size and elastic coefficient of the spring (330), the air pressure required to discharge the powder filled in the powder reservoir (200), etc., unless there is a limitation on the size relationship among them in the present specification.

The plunger (320) can be disposed inside the powder reservoir (200) and can move in the third direction (Z) inside the first sub-body (210) by the third direction (Z) elastic force of the spring (330). The plunger (320) may comprise a packing member (321) and a connecting member (322).

The packing member (321) can seal the other end of the third direction (Z) of the first sub-body (210). The packing member (321) may have a planar shape that is substantially the same as the X-Y plane shape of the first sub-body (210). For example, when the first sub-body (210) has a circular shape in the X-Y plane, the packing member (321) can have a circular shape having a diameter corresponding to the inner diameter of the first sub-body (210). The packing member (321) can have a sealing performance to the extent that foreign substances such as air or dust are not introduced from the opposite direction of the third direction (Z) into the powder filled in the first sub-body (210).

In addition, the packing member (321) can pressurize the powder filled in the first sub-body (210) by the elastic force transmitted from the spring (330), but can have a rigidity that does not deform its shape. For example, the packing member (321) may be made of silicon.

The connecting member (322) may have a structure for connecting the packing member (321) to the spring (330). For example, if the packing member (321) is implemented as a circular ring having a circular shape when viewed in the third direction (Z) and including an opening in the center, the connecting member (322) may have a structure for one end fits into the opening of the packing member (321) in the third direction (Z) and for the other end is coupled with the spring (330).

The connecting member (322) may have a function of sealing the powder filled in the first sub-body (210) similar to the packing member (321). The connecting member (322) may be composed of substantially the same material as the packing member (321), but is not limited thereto.

The packing member (321) and the connecting member (322) may be formed as separate components and may be combined, but are not limited thereto, and the packing member (321) and the connecting member (322) may be formed as one piece.

The spring (330) is arranged to be elastic in the third direction (Z), and one end is fixedly connected to the plunger (320) in the third direction (Z), and the other end can be fixed to the support structure (123) of the second main body (120) or separated from the support structure (123).

The elastic coefficient of the spring (330) can be set so that no empty space is created inside the first sub-body (210) when the plunger (320) pressurizes the powder filled in the powder reservoir (200), as understood by those skilled in the art according to the teachings of the present invention. In addition, the diameter, length and thickness of the spring (330) can be determined according to the elastic coefficient and design details of other components (e.g., the width in the third direction (Z) of the first and second main bodies (110, 120) and the first and second sub-bodies (210, 310)).

The filter (400) may be disposed between the first main body (110) and the first sub-body (210). The filter (400) may be disposed between the outer surface of the first sub-body (210) and the inner surface of the first main body (110), and one end in the third direction (Z) may be in contact with the inner side of one end of the first main body (110). In addition, the filter (400) may be disposed corresponding to the air supply passage (230).

The filter (400) may have a circular ring shape having a width (i.e., width in the first direction (X) and second direction (Y) planes) that is substantially the same as the distance between the outer surface of the first sub-body (210) and the inner surface of the first main body (110). In addition, the third direction (Z) width of the filter (400) may have a width that extends from one end of the first sub-body (210) and completely covers the air supply passage (230).

When an external force is applied to the other side of the second main body (120), and the volume of air placed in the air reservoir (100) decreases and air pressure is generated, the filter (400) allows the air filled in the air reservoir (100) to be supplied to the powder reservoir (200) through the air supply passage (230), but can block the powder filled in the powder reservoir (200) from flowing into the air reservoir (100). As a non-limiting example, the filter (400) may be implemented as a porous filter.

In another embodiment, the shape of the filter (400) may be variously modified according to the number and shape of openings included in the air supply passage (230) within a range that allows the flow of air through the air supply passage (230) between the powder reservoir (200) and the air reservoir (100) but blocks the flow of powder.

The discharge port (500) may comprise a powder discharge passage for discharging powder filled in the powder reservoir (200). The discharge port (500) may be disposed between the spray member (600) or barrier member (700) and the air reservoir (100) or powder reservoir (200).

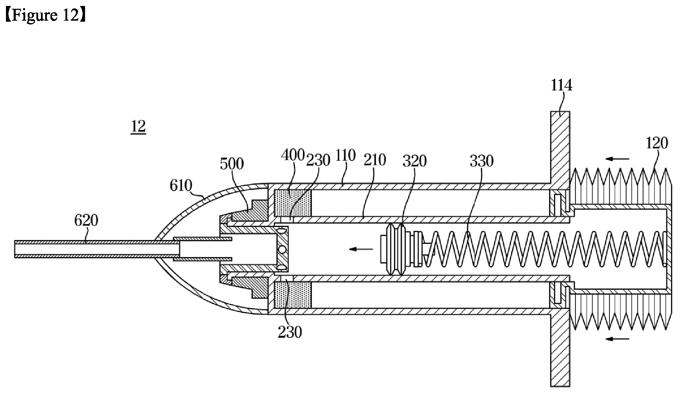

The discharge port (500) may have one end connected to the spray member (600) or the barrier member (700) in the third direction (Z), and the other end connected to the air reservoir (100) and the powder reservoir (200). Specifically, the other end of the discharge port (500) may be coupled and/or connected to the first coupling portion (112) and the second coupling portion (220). With respect to the structure of the discharge port, any known structure may be employed, and for example, reference may be made to the powder delivery device disclosed in Figs. 12 to 14 of U.S. Patent No. 10507293, but is not limited thereto.

The discharge port (500) may comprise an internal structure and an external structure. The internal structure may have a cylindrical structure in which an outer surface is fitted to an inner surface of the second coupling portion (220) and a powder discharge passage is formed inside. The external structure may be formed on the outside of the internal structure and may comprise an inner surface for coupling with the first coupling portion (112) and an outer surface for coupling with the spray member (600) or the barrier member (700). The discharge port (500) may have a shape in which the internal structure protrudes in the opposite direction of the third direction (Z) compared to the external structure. The internal structure and the external structure of the discharge port (500) may be changed according to the structures of the first coupling portion (112) and the second coupling portion (220).

The discharge port (500) may comprise a powder trap (PT). The powder trap (PT) may be formed at the other end of the internal structure in the third direction (Z). A separate powder trap member (PTM) may be coupled to the other end of the internal structure to form the powder trap (PT), but is not limited thereto. For example, through a technology such as 3D printing, the powder trap (PT) may be formed integrally with the discharge port (500).

When the powder discharge passage of the discharge port (500) is directly connected to the internal space of the first sub-body (210) of the powder reservoir (200), the powder filled in the powder reservoir (200) can be discharged as one end of the discharge port (500) faces the direction of gravity. Accordingly, the powder trap (PT) may have a structure for preventing discharge of powder other than powder filled in the powder reservoir (200) being intentionally discharged by air pressure.

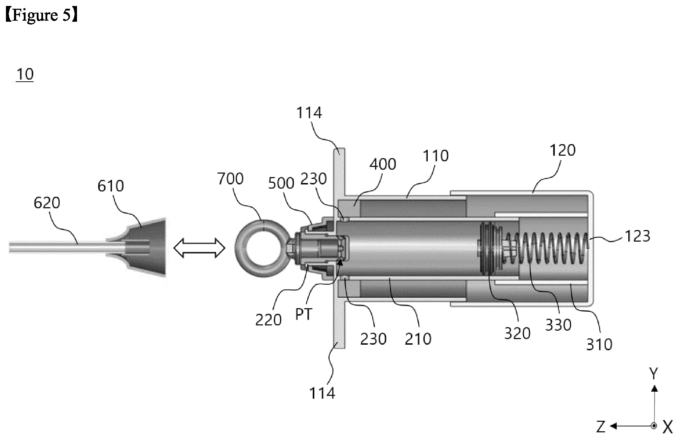

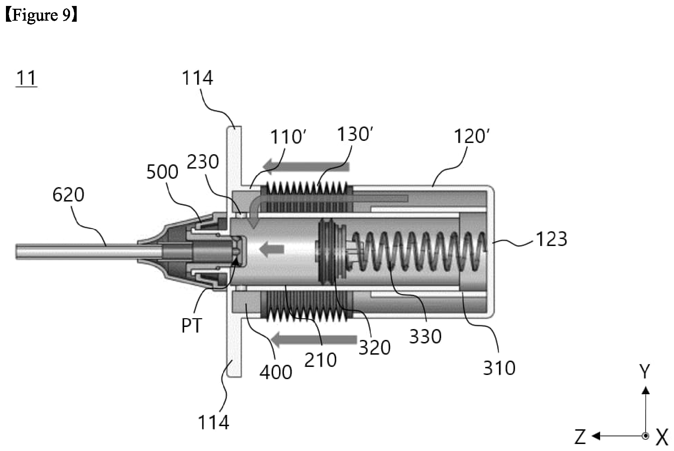

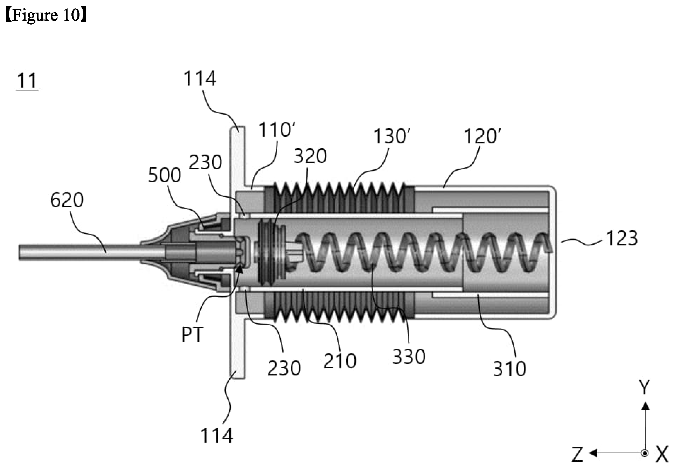

For example, the powder trap (PT) may comprise a passage in the shape of an arrow facing in the opposite direction to the direction in which the powder is discharged (the third direction (Z)), as illustrated in Figs. 5 to 10, but may comprise a passage having a blunt cross-sectional shape at the end of the arrow. That is, the passage of the powder trap (PT) may have a path in which the powder reservoir (200) and the discharge port (500) are connected, but a portion adjacent to the powder reservoir (200) faces in the opposite direction to the direction in which the powder is discharged by the discharge port (500) (i.e., in the opposite direction to the third direction (Z)).

Accordingly, even if the discharge port (500) is arranged along the direction in which gravity acts, the powder filled in the powder reservoir (200) cannot flow into the passage of the powder trap (PT) facing in the opposite direction of gravity, and thus, in addition to the powder being intentionally discharged when an external force is applied to the other end of the powder applicator (10), the powder can be prevented from being unintentionally discharged.

In the present specification, it is exemplified that there are two passages interconnecting with the powder trap (PT) and the powder reservoir (200), but this is not limited thereto. In another example, there may be three or more passages interconnecting with the powder trap (PT) and the powder reservoir (200), or there may be only one.

A third sealing member (PM3) may be disposed on at least a portion of the outer surface of the inner structure. The third sealing member (PM3) may provide airtightness between the inner structure of the discharge port (500) and the second coupling portion (220) of the powder reservoir (200).

The spray member (600) is configured to be coupled to the discharge port (500) to inject powder into the affected area, and may comprise a needle (610) and a needle tube (620). The needle (610) is coupled to the discharge port (500), and the needle tube (620) may be connected to the powder discharge passage of the discharge port (500) through the needle (610).

The shape of the spray member (600) may be applied to various types of spray members that are understood by those skilled in the art. For example, the spray member (600) may have various structures depending on the type and size of the affected area where the powder is sprayed, such as having a structure suitable for insertion into the patient's body through an endoscope during surgery.

The barrier member (700) may be combined with the discharge port (500) instead of the spray member (600) before the powder applicator (10) is used. The barrier member (700) can prevent the powder filled in the powder reservoir (200) from being exposed to the outside air or foreign substances such as dust from flowing into the powder reservoir (200) through the discharge port (500).

The barrier member (700) may comprise a cylindrical structure for blocking the powder discharge passage of the discharge port (500). In addition, the barrier member (700) may comprise a structure that connects to the cylindrical structure and is advantageous for the barrier member (700) to be separated from the discharge port (500). For example, the barrier member (700) may have a structure in which a cylindrical structure and a ring-shaped structure are connected in a third direction (Z), but is not limited thereto. As another example, the barrier member (700) may have various structures, such as a cylindrical structure and a hook-shaped structure connected in the third direction (Z) or a cylindrical structure and a 'T'-shaped structure connected.

A fifth sealing member (PM4) in the shape of an O-ring may be disposed between the spray member (600) or barrier member (700) and the discharge port (500) to ensure airtightness between the two components.

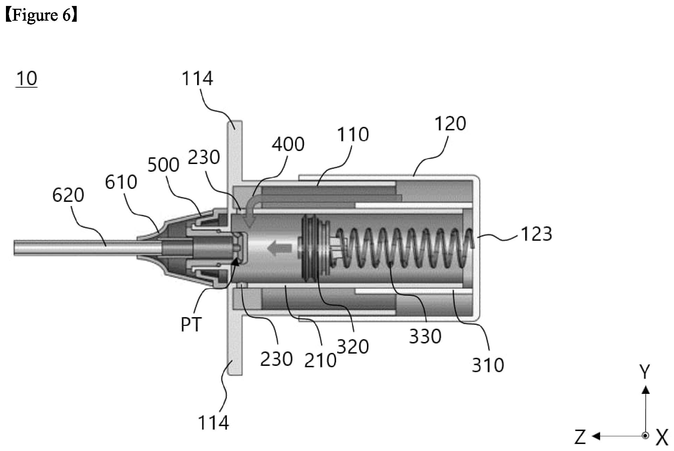

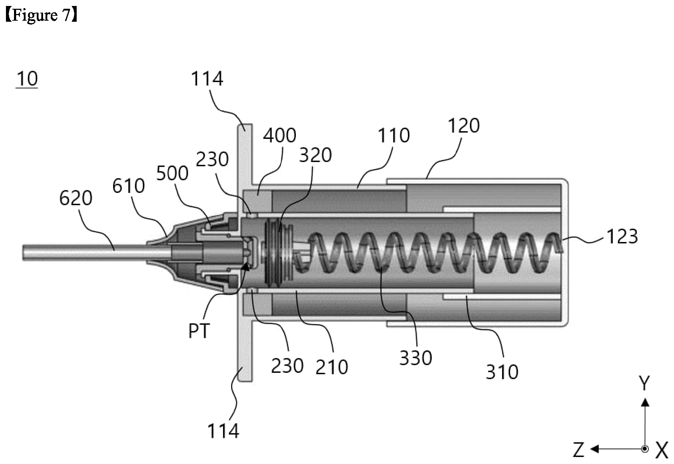

Figs. 5 to 7 are cross-sectional views of the powder applicator taken along line L-L' of Fig. 1, wherein Fig. 5 is a drawing showing the powder applicator (10) in a state where the airtightness of the powder is maintained by a barrier member (700) before the powder applicator (10) is used, Fig. 6 is a drawing showing a process in which powder filled in the powder applicator (10) is discharged as an external force is applied to the air reservoir (100) and the operating unit (300), and Fig. 7 is a drawing showing the powder applicator (10) after all powder filled in the powder reservoir (200) is discharged.

Hereinafter, a method of operating a powder applicator (10) according to one embodiment will be described with reference to Figs. 5 to 7. In Figs. 5 to 6, the powder filled in the powder reservoir (211) is omitted for convenience of explanation, but powder may be filled between the plunger (320) and the powder trap (PT).

First, referring to Fig. 5, a powder applicator (10) according to one embodiment may have a barrier member (700) coupled to a discharge port (500) to ensure the airtightness of powder filled in a powder reservoir (200) before being used by the user. In addition, before the powder applicator (10) is used, a spring (330) may be in a compressed state with a length shorter than the length of the equilibrium state of the spring. As described below, as powder is discharged by the powder applicator (10), the compressive force applied to the spring (330) decreases, and a third direction (Z) length of the spring (330) may become longer.

Thereafter, for use, the user can separate the barrier member (700) from the discharge port (500) and attach a spray member (600) suitable for the area where the powder is to be applied to the discharge port (500).

In this case, even if one end of the discharge port (500) is opened to the outside as the spray member (600) is coupled to the discharge port (500), the powder applicator (10) according to one embodiment can prevent powder from being unintentionally discharged from the powder applicator (10) in a situation where the discharge port (500) is oriented in the direction of gravity or an external action such as vibration is applied to the powder applicator (10), since the discharge port (500) includes a powder trap (PT).

Next, the user can use the handle portion (114) to support the powder applicator (10) from moving in the third direction (Z) and apply an external force to the other end of the second main body (120) to spray the powder filled in the powder reservoir (200).

For example, a user may support one end surface of the handle portion (114) using the index and middle fingers of one hand and press the other end of the second main body (120) with the thumb of the same hand. Accordingly, as illustrated in Figs. 5 and 6, in the powder applicator (10), the second main body (120) and the second sub-body (310) can slide and move in the third direction (Z) relative to the first main body (110) and the first sub-body (210), respectively. The air filled in the internal space of the air reservoir (100) is compressed, and the compressed air may pass through the filter (400) and be supplied to the inside of the powder reservoir (200) through the air supply passage (230).

The powder filled in the powder reservoir (200) can be transported by the airflow supplied through the air supply passage (230) and applied to the patient's affected area through the discharge port (500) and spray member (600) via the powder trap (PT).

In this way, since the powder applicator (10) according to the present invention allows the airflow for transporting the powder to be introduced through the air supply passage (230) disposed adjacent to the location where the discharge port (500) and the powder trap (PT) are disposed, the power for moving the powder filled in the powder reservoir (200) to the discharge port (500) through the powder trap (PT) can be efficiently transmitted, thereby increasing the flowability of the powder and being advantageous in effectively discharging the powder.

In addition, when the airflow for discharging the powder flows into the other end of the powder reservoir (200), for example, into the part where the powder is pressurized by the plunger (320), the air flowing into the powder reservoir (200) is resisted by the other end of the powder trap (PT), whereas the powder applicator (10) of the present invention prevents air resistance by the powder trap (PT) and at the same time separates the direction of air inflow from the direction where the powder is pressurized by the plunger (320), thereby being advantageous in more efficiently discharging the powder filled in the powder reservoir (200).

Meanwhile, since the powder applicator (10) according to one embodiment is disposed so that the first main body (110) and the second main body (120) and the first sub-body (210) and the second sub-body (310) are each at least partially overlapped and fitted and slide in the third direction (Z) as illustrated in Figs. 5 to 10, the powder applicator (10) may be pressed in a constant direction regardless of the position of the finger pressing the other end of the second main body (120) (i.e., the position of the user's thumb on the other end surface of the second main body (120) in the X-Y plane) and the angle at which the other end of the second main body (120) is pressed.

Therefore, in the case of the powder applicator (10) according to one embodiment, since a uniform pressure can be applied regardless of the location where the user applies pressure to the powder applicator (10) to spray the powder, it can be advantageous to operate the powder applicator (10) to discharge a constant amount of powder.

In addition, as the second main body (120) and the second sub-body (310) move in the third direction (Z) with respect to the first main body (110) and the first sub-body (210), respectively, the plunger (320) and the spring (330) also move in the third direction (Z), and the plunger (320) may move by the amount of space occupied by the powder discharged from the powder reservoir (200) due to the external force applied to the second main body (120) and the elastic force of the spring (330).

When the powder applicator (10) is designed to spray a plurality of times, the powder reservoir (200) can be maintained in a state filled with powder without any empty space by the plunger (320) before all of the powder filled in the powder reservoir (200) is sprayed. Therefore, in the case of the powder applicator (10) according to one embodiment, it is advantageous to maintain a constant amount of powder to be sprayed according to the plurality of times and to improve the accuracy of the timing at which the powder is sprayed.

When the external force applied to the other end of the second main body (120) is released, the second main body (120) and the second sub-body (310) may move in the opposite direction of the third direction (Z) with respect to the first main body (110) and the first sub-body (210) by the elastic force of the spring, respectively, and can be restored to the original position before the external force was applied to the second main body (120). Even in this case, the plunger (320) can still be maintained without a change in position in the internal space of the powder reservoir (200) so that the powder reservoir (200) is filled with powder without any empty space therein. In addition, since the filter (400) is disposed between the air reservoir (100) and the powder reservoir (200), the movement of powder from the powder reservoir (200) to the air reservoir (100) can be blocked.

In the powder applicator (10), the above-described powder discharging process can be repeated a preset number of times. When the powder applicator (10) discharges all of the filled powder, the plunger (320) can come into contact with one end surface of the internal space of the powder reservoir (200), as shown in Fig. 7. To this end, the elastic coefficient of the spring (330) can be designed to have an elasticity that allows the plunger (320) to come into contact with one end surface of the internal space of the powder reservoir (200) as the powder filled in the powder applicator (10) is discharged.

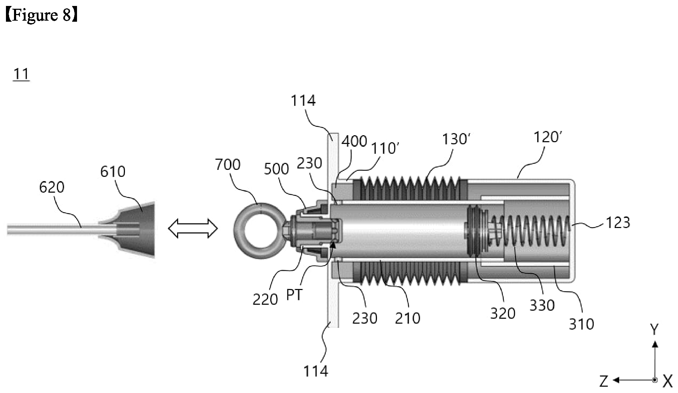

Figs. 8 to 10 are cross-sectional views for explaining the operation method of a powder applicator according to another embodiment of the present invention.

The powder applicator (11) according to Figs. 8 to 10 differs from the embodiment of Figs. 5 to 7 in that the air reservoir (100) further comprises a bellows member (130'). With respect to Figs. 8 to 10, the differences from the embodiments of Figs. 5 to 7 will be mainly explained.

Referring to Figs. 8 to 10, a bellows member (130') of a powder applicator (11) according to another embodiment of the present invention can be disposed between the first main body (110') and the second main body (120').

The bellows member (130') may have a structure that folds in a corrugated manner when force is applied in the third direction (Z). The bellows member (130') may have elasticity in the third direction (Z). For example, before an external force is applied to the powder applicator (11), the bellows member (130') has an elastic equilibrium state, but when an external force is applied in the third direction (Z), it may have elasticity to return to the elastic equilibrium state.

The first main body (110') according to the present embodiment may have a smaller width in the third direction (Z) than the first main body (110) according to the embodiments of Figs. 5 to 7. In another embodiment derived from the present embodiment, the width in the third direction (Z) of the second main body (120') may be smaller than the width in the third direction (Z) of the second main body (120) according to the embodiments of Figs. 5 to 7, but is not limited thereto.

The first main body (110') and the second main body (120') may have substantially the same diameter and may not overlap in the X-Y plane. In this case, one end and the other end of the bellows member (130') in the third direction (Z) may have diameters for being coupled and fixed to the first main body (110') and the second main body (120'), respectively. As another example, the first main body (110') and the second main body (120') may have different diameters, and the bellows member (130') may also have diameters for being coupled and fixed to the first main body (110') and the second main body (120'), respectively. Accordingly, the bellows member (130') may have a non-uniform width in the second direction (Y) in the third direction (Z).

When an external force is applied by a user in a third direction (Z) to the powder applicator (11) according to Figs. 8 to 10, the structure that folds in a corrugated manner of the bellows member (130') is folded, and the first main body (110') and the second main body (120') may be moved to be adjacent to each other in the third direction (Z). Accordingly, the pressure of the air filled inside the air reservoir (100') increases, and as examined with reference to Figs. 5 to 7, the air with increased pressure may be introduced into the powder reservoir (200) through the air supply passage (230) to transport the powder to the powder trap (PT) and the discharge port (500).

In the case of the powder applicator (11) according to the embodiments of Figs. 8 to 10, it can be advantageous to control the amount of powder sprayed to the powder applicator (11) by controlling the elasticity of the bellows member (130') in the third direction (Z). In addition, similar to the embodiments of Figs. 5 to 7, when a force in the third direction (Z) is applied to the powder applicator (11), since the first sub-body (210) and the second sub-body (310) slide toward each other in the third direction (Z) while at least partially overlapping and fitting together, the pressure can be applied in a constant direction regardless of the position of the user's finger pressing the other end of the second main body (120) and the angle at which the other end of the second main body (120) is pressed.

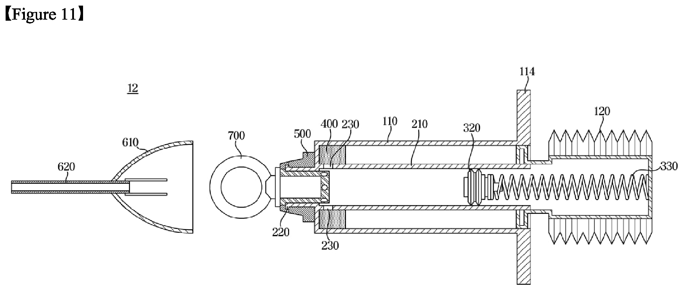

Figs. 11 to 13 are cross-sectional views for explaining an operating method of a powder applicator (12) according to another embodiment of the present invention. The powder applicator (12) may be implemented by omitting the second sub-body (310) from the operating unit (300) and implementing the second body portion of the second main body (120) as a bellows member, and may have, for example, the same operating principle as the powder applicator of Figs. 14 to 17.

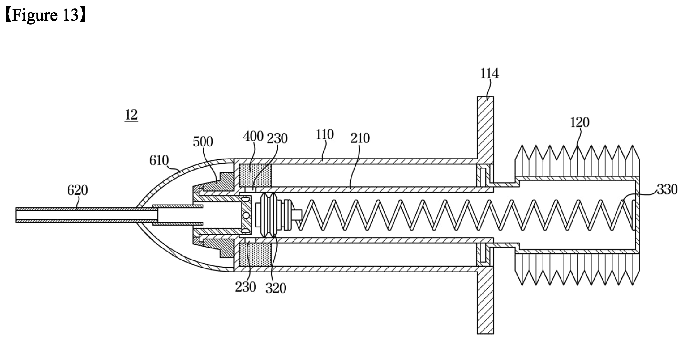

Fig. 11 is a drawing showing a powder applicator (12) in a state in which the airtightness of the powder is maintained by a barrier member (700) before the powder applicator (12) is used, Fig. 12 is a drawing showing a process in which powder filled in the powder applicator (12) is discharged as an external force is applied to an air reservoir (100) and an operating unit (300), and Fig. 13 is a drawing showing the powder applicator (12) after all powder filled in the powder reservoir (200) is discharged.

Referring to Figs. 11 to 13, the second main body (120) according to the present embodiment may have the second body portion formed as a bellows member. For example, the second body portion may be a bellows member having a shape extending from the other end of the first body portion in the third direction (Z). Accordingly, the second stopping portion (122) of the second main body (120) of the embodiments of Figs. 5 to 7 may be omitted in the present embodiment. The second body portion may be a bellows member having a constant width in the third direction (Z), but may also be formed in a tapered shape in which the width in the third direction (Z) changes, as illustrated in Fig. 14.

In addition, the first main body (110) may be entirely transparent or partially transparent, and the first main body (110) may have a constant diameter or a tapered shape formed so that the width in the third direction (Z) changes. In addition, the handle portion (114) may be symmetrical or asymmetrical, and may have a structure that protrudes outwardly from the first main body (110) or may be formed as a separate structure from the first main body (110) and coupled to one end of the first main body (110).

A spring (330) may be fixed or spaced on the inner side of the second body portion of the above bellows member shape.

Hereinafter, a method of operating a powder applicator (12) according to one embodiment will be described with reference to Figs. 11 to 13. In Figs. 11 and 12, the powder filled in the powder reservoir (211) is omitted for convenience of explanation, but the space between the plunger (320) and the powder trap may be filled with powder.

First, referring to Fig. 11, a powder applicator (12) according to one embodiment may have a barrier member (700) coupled to a discharge port (500) to ensure the airtightness of powder filled in a powder reservoir (200) before being used by a user. In addition, before the powder applicator (12) is used, a spring (330) may be in a compressed state with a length shorter than the length of the equilibrium state of the spring. As described below, as powder is discharged by the powder applicator (12), the compressive force applied to the spring (330) decreases, and a third direction (Z) length of the spring (330) may become longer.

Before the powder applicator (12) according to another embodiment is used by a user, the spring (330) may be spaced from the inside of the second main body (120) in the shape of a bellows member and may be in an uncompressed equilibrium state, and when an external force is applied to the other end of the second main body (120), the spring (330) may be compressed and move forward in the third direction (Z), and when the external force applied to the other end of the second main body (120) is released, the compressive force applied to the spring (330) may be reduced and may be restored to the length of the original equilibrium state.

Thereafter, for use, the user can separate the barrier member (700) from the discharge port (500) and attach a spray member (600) suitable for the area where the powder is to be applied to the discharge port (500).

In this case, even if one end of the discharge port (500) is opened to the outside as the spray member (600) is coupled to the discharge port (500), the powder applicator (12) according to one embodiment can prevent powder from being unintentionally discharged from the powder applicator (12) in a situation where the discharge port (500) faces the direction in which gravity acts or an external action such as vibration is applied to the powder applicator (12), since the discharge port (500) includes a powder trap.

Next, the user can use the handle portion (114) to support the powder applicator (12) from moving in the third direction (Z) and apply an external force to the other end of the bellows member, which is the second main body (120), to spray the powder filled in the powder reservoir (200).

For example, a user may support one end surface of the handle portion (114) using the index and middle fingers of one hand and press the other end of the bellows member, which is the second main body (120), with the thumb of the same hand. Accordingly, in the powder applicator (12) as illustrated in FIGS. 11 and 12, the bellows member, which is the second main body (120), is contracted toward the first main body (110), the air filled in the air reservoir (100) is compressed by increasing pressure, and the compressed air can be supplied into the powder reservoir (200) through the air supply passage (230) via the filter (400). The plunger (320) can pressurize the powder filled in the powder reservoir (200).

The powder filled in the powder reservoir (200) may be transported by the airflow supplied through the air supply passage (230) and applied to the patient's affected area through the discharge port (500) and spray member (600) via the powder trap.

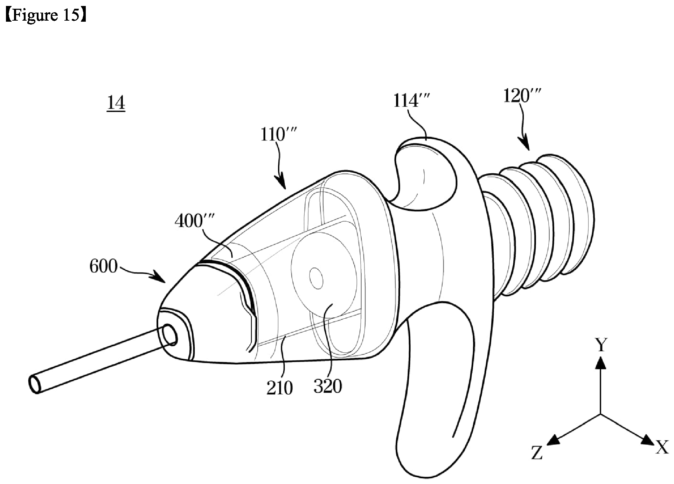

The powder applicator of the present invention is not limited to the specific examples described and illustrated above, and may be formed by mixing the configurations described in each specific example. For example, a powder applicator according to another embodiment may be basically the same as the embodiment of Fig. 14, and the shape of the handle portion and/or the shape of the second body portion may be the same as those illustrated in Figs. 15 to 17. In addition, according to another embodiment, the plunger (320) may be formed by integrally forming the packing member and the connecting member.

Fig. 14 is a schematic perspective view of a powder applicator (13) according to another embodiment of the present invention.

The embodiment of Fig. 14 differs from the embodiments of Figs. 5 to 7 in that the position and shape in which the handle portion (114") of the first main body (110") is formed and the second body portion of the second main body (120") is implemented as a bellows member. With respect to Fig. 14, the differences from the embodiments of Figs. 5 to 7 will be mainly described.

Referring to Fig. 14, the second main body (120") according to the present embodiment may have the second body portion formed as a bellows member. For example, the second body portion may be a bellows member having a shape extending from the other end of the first body portion in the third direction (Z). Accordingly, the second stopping portion (122) of the second main body (120) of the embodiments of Figs. 5 to 7 may be omitted in the present embodiment. The second body portion may be a bellows member having a constant width in the third direction (Z), but may be formed such that the width in the third direction (Z) varies, as illustrated in Fig. 14.

A spring (330) may be fixed or spaced on the inner side of the second body portion of the above bellows member shape.

The handle portion (114") of the first main body (110") may be formed at the other end of the first body portion in the third direction (Z). For example, when the second body portion formed as a bellows member by extending from the other end of the first body portion is operated, the handle portion (114") may have a shape in which at least a portion of the circumference of the other end of the first body portion protrudes laterally for power transmission and stability. The degree and shape of the handle portion (114") protruding from the other end of the first body portion may be designed to be variously changed. For example, the handle portion (114") may protrude from the side of the other end of the first body portion in the second direction (Y) and in the opposite direction of the second direction (Y), respectively, and may have a shape of the number 1 crossing the first body portion when viewed in the third direction (Z), and the portions protruding in the second direction (Y) and in the opposite direction of the second direction (Y), respectively, may be symmetrical or asymmetrical to each other.

Meanwhile, in the case of the present embodiment, the operating unit (300) may be implemented by omitting the second sub-body (310).

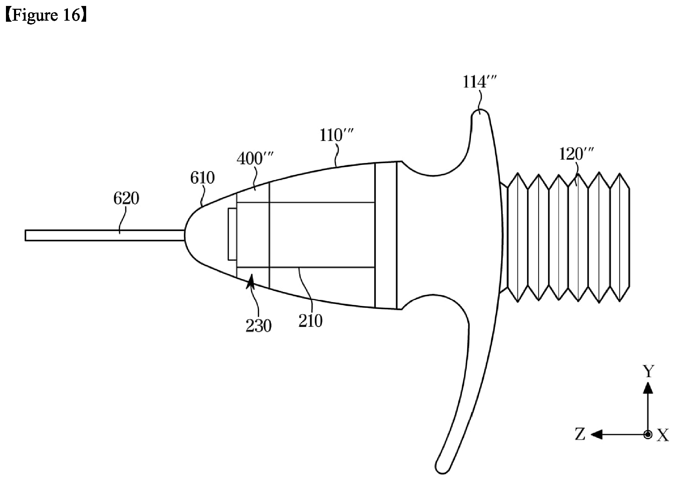

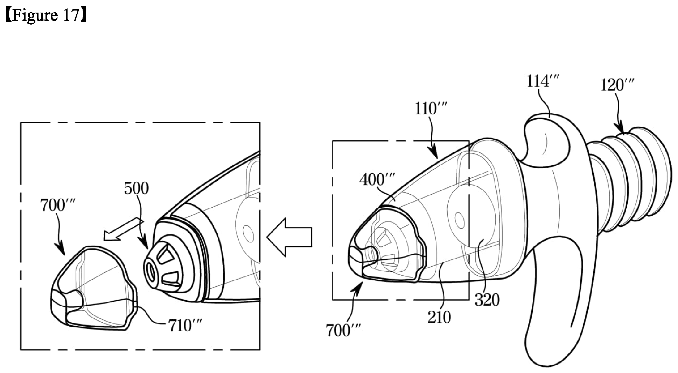

Fig. 15 is a schematic perspective view of a powder applicator (14) according to another embodiment of the present invention. Fig. 16 is a cross-sectional view of the powder applicator (14) of Fig. 15. Fig. 17 is a perspective view showing an example in which a barrier member (700‴) according to another embodiment of the present invention is applied to the powder applicator (14) of Fig. 15.

The embodiments of Figs. 15 to 17 differ from the embodiment of Fig. 14 in that the shape of the first body portion of the first main body (110‴), the shape of the handle portion (114‴), the shape of the filter (400‴), and the shape of the barrier member (700‴) are modified, and the second body portion of the second main body (120‴) is formed as a bellows member having a constant width in the third direction (Z). With respect to Figs. 15 to 17, the differences from the embodiment of Fig. 14 will be mainly described.

First, referring to Figs. 15 and 16, the first body portion of the first main body (110‴) according to the present embodiment may have a shape whose width changes in the third direction (Z). For example, the first body portion may have a shape whose area on the X-Y plane increases from one end to the other end, and although not limited thereto, when viewed in the first direction (X), the first body portion may have a triangular shape on the plane. In this case, when air is supplied to the powder reservoir (200) through the air supply passage (230) from the air reservoir (100‴) by air pressure, since the air reservoir (100‴) has a structure in which the area of one end side is smaller than that of the other end side in the third direction (Z), it may be advantageous in improving the air supply efficiency. Additionally, the filter (400‴) may have a shape that can be disposed inside the first main body (110"') having such a shape.

In addition, the handle portion (114‴) may be implemented so that the overall shape of the powder applicator (13) has a T-shape or a gun shape. The handle portion (114‴) according to the present embodiment may be advantageous in improving the stability of the operation of the powder applicator (14) by improving the grip and supportability when the powder applicator (14) is used by a user.

Referring to Fig. 17, the barrier member (700‴) may have a shape that closes the discharge port (500) and provides a sense of unity with the first body portion. For example, the area of the other end of the barrier member (700‴) on the X-Y plane in the third direction (Z) is substantially the same as the area of the one end of the first body portion on the X-Y plane, and the barrier member (700‴) may have a shape in which the area on the plane decreases as it goes in the third direction (Z).

In addition, a separation structure (710‴) may be formed on at least a portion of the other end of the barrier member (700‴) so that the barrier member (700‴) can be easily separated from the discharge port (500). The separation structure (710‴) may have a shape in which at least a portion of the other end of the barrier member (700‴) protrudes laterally on the X-Y plane, but is not limited thereto.

Even in the case of the embodiments according to Figs. 14 to 17, since the airflow for transporting the powder is introduced through the air supply passage (230) disposed adjacent to the location where the discharge port (500) and the powder trap (PT) are disposed, the force for moving the powder filled in the powder reservoir (200) to the discharge port (500) through the powder trap (PT) can be efficiently transmitted, and accordingly, the flowability of the powder can be increased, which can be advantageous in effectively discharging the powder.

In addition, when the airflow for discharging the powder flows into the other end of the powder reservoir (200), for example, into the part where the powder is pressurized by the plunger (320), the air flowing into the powder reservoir (200) is resisted by the other end of the powder trap (PT), whereas the powder applicator (13, 14) according to the embodiments can prevent air resistance by the powder trap (PT) and at the same time, separate the direction of air inflow from the direction where the powder is pressurized by the plunger (320), thereby being advantageous in more efficiently discharging the powder filled in the powder reservoir (200).

Although the embodiments of the present invention have been described with reference to the attached drawings, it will be understood by those skilled in the art that the present invention may be implemented in other specific forms without changing the technical spirit or essential features of the present invention. These embodiments are not intended to limit the invention but are merely illustrative, and should be considered from an illustrative rather than a restrictive perspective. Although specific terms have been used in this specification, they have been used only for the purpose of explaining the concept of the present invention and have not been used to limit the scope of the present invention as set forth in the claims or in the meaning thereof. Therefore, the embodiments described above should be understood as illustrative and not restrictive in all respects.

## Claims

1. A powder applicator comprising,
an air reservoir comprising a first main body and a second main body interconnected with each other, forming the outline of a powder applicator, and configured to contain air;
a powder reservoir comprising a first sub-body and an air supply passage, wherein the first sub-body is disposed between a discharge port through which powder is discharged and an operating unit, is disposed inside the first main body, and includes a space configured to be filled with powder, and wherein the air supply passage is formed in a portion adjacent to the discharge port of the first sub-body and includes at least one opening;
a filter disposed between the powder reservoir and the air reservoir so as to correspond to the air supply passage, wherein the filter is configured to block movement of powder between the powder reservoir and the air reservoir and to allow movement of air; and
an operating unit including a plunger disposed inside the powder reservoir and dividing the space of the first sub-body.

2. The powder applicator according to claim 1,
wherein the operating unit further comprises a second sub-body that at least partially overlaps the powder reservoir and is slidably disposed with respect to the powder reservoir.

3. The powder applicator according to claim 1 or 2,
wherein the operating unit further comprises a spring disposed between the plunger and one end of the first sub-body to exert pressure on powder contained in the powder reservoir through the plunger.

4. The powder applicator according to claim 3,
wherein the spring has an elastic force for the plunger to be in close contact with one end surface of the internal space of the powder reservoir.

5. The powder applicator according to claim 3,
wherein the plunger is pressurized by the spring to pressurize the powder.

6. The powder applicator according to claim 1 or 2,
further comprising a barrier member coupled to the discharge port to block contact between the powder and the external environment.

7. The powder applicator according to claim 1 or 2,
wherein the air reservoir further comprises a handle portion having a structure protruding outwardly from the first main body or a handle portion formed as a separate structure from the first main body and coupled to one end of the first main body.

8. The powder applicator according to claim 1,
wherein, when an external force is applied to the second main body in one direction, the second main body contracts toward the first main body, the air filled in the air reservoir increases in pressure and is supplied to the powder reservoir through the air supply passage, and the plunger pressurizes the powder filled in the powder reservoir.

9. The powder applicator according to claim 1,
wherein the first main body and the second main body are disposed to at least partially overlap, and wherein the second main body is in the form of a bellows member having a corrugated folding structure.

10. The powder applicator according to claim 2,
wherein when an external force is applied to the second main body in one direction, the second main body and the second sub-body move toward the first main body and the first sub-body, respectively, and the air filled in the air reservoir increases in pressure so as to be supplied to the powder reservoir through the air supply passage, and the plunger pressurizes the powder filled in the powder reservoir.

11. The powder applicator according to claim 2,
wherein the first main body and the second main body are disposed to at least partially overlap, wherein each of the first main body and the second main body comprise a first stopping portion and a second stopping portion that are mutually engaged at end sides adjacent to each other, and the first main body and the second main body are slidable relative to each other.

12. The powder applicator according to claim 2,
wherein the air reservoir further comprises a bellows member, and
the bellows member is disposed between the first main body and the second main body and has a structure that folds in a corrugated manner in one direction.
